(19) Europäisches Patentamt
European Patent Office
Office européen des brevets

(11) EP 2 662 081 A1

# (12) EUROPEAN PATENT APPLICATION

(43) Date of publication:
**13.11.2013 Bulletin 2013/46**

(21) Application number: **12167268.7**

(22) Date of filing: **09.05.2012**

(51) Int Cl.:
***A61K 31/4178*** *(2006.01)*
***A61P 9/00*** *(2006.01)*
***A61P 3/10*** *(2006.01)*
***A61P 25/00*** *(2006.01)*
***A61P 29/00*** *(2006.01)*
***A61P 31/12*** *(2006.01)*
***A61P 37/02*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
- **NOSCIRA, S.A.**
  **28760 Tres Cantos, Madrid (ES)**
- **Smith, Andrew**
  **Brisbane, Queensland 4072 (AU)**

(72) Inventors:
- **Medina Padilla, Miguel**
  **E-28760 Tres Cantos - Madrid (ES)**
- **Orozco Muñoz, Leyre**
  **E-28760 Tres Cantos - Madrid (ES)**
- **Capon, Robert**
  **Brisbane, Queensland 4072 (AU)**

(74) Representative: **ABG Patentes, S.L.**
**Avenida de Burgos, 16D**
**Edificio Euromor**
**28036 Madrid (ES)**

## (54) Therapeutic use of indole-dihydro-imidazole derivatives

(57) The present invention is related to the use of indole-dihydro-imidazole derivatives of Formula (I) as a medicament for the treatment of diseases or conditions mediated by Cdk5 and/or GSK3.

EP 2 662 081 A1

**Description**

## FIELD OF THE INVENTION

The present invention is related to the use of indole-dihydro-imidazole derivatives of Formula (I) as a medicament for the treatment of diseases or conditions mediated by Cdk5 and/or GSK3.

## BACKGROUND OF THE INVENTION

### *Cyclin-dependent kinase 5 (Cdk5)*

Cyclin-dependent kinase 5 (Cdk5) is a proline-directed serine/threonine protein kinase with a growing number of identified substrates, such as β-Amyloid precursor protein (β-APP), presenilin-1, tau protein or neurofilaments (NFs) [Nat. Rev., Mol. Cell Biol., 2001, 2, 749-759. A decade of CDK5. Dhavan R. & Tsai L.H.; Journal of Neurochemistry, 2004, 88, 1313-1326. Cyclin-dependent kinase-5 in neurodegeneration. Shelton S.B. & Johnson G.V.W; Cell Mol Neurobiol, 2008, 28, 351-369. An Unusual Member of the Cdk Family: Cdk5. *Dhariwala F.A. & Rajadhyaksha M.S*.]. Cdk5 does not play a role in cell division but plays a pivotal role in the development of the central nervous system (CNS) [Nat. Rev., Mol. Cell Biol., 2001, 2, 749-759. A decade of CDK5. Dhavan R. & Tsai L.H.]. Cdk5 functions beyond neurodevelopment and may play a role in learning and memory, in dopamine signaling in the basal forebrain, in the migration and diferentiation of neurons during histogenesis of the brain, in regulation of the cytoskeletal elements, membrane transport, synaptic function and during neuronal differentiation and axonal extension. Therefore, CDK5 likely plays a role in synaptic plasticity, cellular motility and adhesion, drug addiction and neurodegeneration [Biochimica et Biophysica Acta 1697 (2004) 137- 142. Cdk5, a therapeutic target for Alzheimer's disease?. Tsai L.H., Lee M.S. & Cruz J; Cell Mol Neurobiol, 2008, 28, 351-369. An Unusual Member of the Cdk Family: Cdk5. Dhariwala F.A. & Rajadhyaksha M.S. *;* J. Comp. Neurol., 1999, 415, 218-229. Callosal axon guidance defects in p35 (-/-) mice. Kwon Y. T., Tsai L.H. & Crandall J. E.; Journal of Neurochemistry, 2004, 88, 1313-1326. Cyclin-dependent kinase-5 in neurodegeneration. *Shelton S. B. & Johnson G.V.W*].

In order for Cdk5 to become active, it must form a heterodimer with p35, p25 (the truncated form of p35), p39 or p29 (the truncated form of p39) [Journal of Neurochemistry, 2004, 88, 1313-1326. Cyclin-dependent kinase-5 in neurodegeneration. Shelton S.B. & Johnson G.V.W]. Both p35 and p39 have a short half-life *in vivo* and are subject to ubiquitin-mediated proteosome degradation indicating that Cdk5 activity is normally tightly regulated. Several neurotoxic conditions, including ischemic brain damage, oxidative stress, excitotoxicity and β-amyloid peptide treatment of primary neurons, were shown to induce calpain-mediated p35 cleavage and p25 production. p25, which is neurotoxic, contains all the elements necessary for Cdk5 activation. It efficiently activates Cdk5 both *in vitro* and *in vivo.* p25 and p35 have distinct properties: p35 has a very short half-life but p25 is stable and has a much longer half-life; p35 is localized to the membranes but p25 localizes to the cell soma and nucleus. As such, generation of p25 disrupts the normal regulation of Cdk5 by causing prolonged activation and mislocalization of Cdk5 and resulting in hyperphosphorylation of available substrates [Biochimica et Biophysica Acta 1697 (2004) 137 142. Cdk5, a therapeutic target for Alzheimer's disease?. Tsai L.H., Lee M. S. & Cruz J.].

Although Cdk5 is expressed in most tissues, its activity is found predominantly in post-mitotic neurons [Development, 2003, 119, 1029-1040. Activity and expression pattern of cyclin-dependent kinase 5 in the embryonic mouse nervous system. Tsai L. H. *et al*.]. The reason for the localized activity of Cdk5 is that its activators, p35 and p39, are expressed almost exclusively in the CNS [Nature, 1994, 371, 423-426. A brain-specific activator of cyclin-dependent kinase 5. Lew et al.; Nature, 1994, 371, 419-423. p35 is a neural-specific regulatory subunit of cyclin-dependent kinase 5. Tsai et al.; FEBS Lett., 1994, 355, 35-40. Precursor of cdk5 activator, the 23 kDa subunit of tau protein kinase II: its sequence and developmental change in brain. Uchida et al.; J. Biol. Chem., 1995, 270, 26897-26903. An isoform of the neuronal cyclin-dependent kinase 5 (Cdk5) activator. Tang et al.; Neuroscience, 2003, 118, 323-334. The cyclin-dependent kinase 5 activator, p39, is expressed in stripes in the mouse cerebellum Jeong et al.; Cell. Mol. Biol. Lett., 2003, 8, 546,547. The role of cyclin-dependent kinase 5 in brain development and degeneration Tsai L.H.]. Although Cdk5 is present predominantly in the nervous system, this enzyme has been implicated to be an important molecule in other systems as well. Since its discovery over a decade ago, an increasing number of the Cdk5 substrates have been reported, making it one of the most versatile kinases. Cdk5 is also known to play an important role in other non-neuronal functions, like myogenesis, haematopoesis, reproduction, prostate cancer, differentiation of lens epithelial and corneal cells, gene-transcription, senescence, insulin secretion, apoptosis, exocytosis/endocytosis, hormonal regulation and wound healing and migration [for a review, see Cell Mol Neurobiol, 2008, 28, 351-369. An Unusual Memberii of the Cdk Family: Cdk5. Dhariwala F.A. & Rajadhyaksha M.S.; J. Comp. Neurol., 1999, 415, 218-229. Callosal axon guidance defects in p35 (-/-) mice. *Kwon Y. T., Tsai L.H. & Crandall J. E.*].

The Cdk5 kinase is activated in cultured neurons following p25 overexpression, and leads to tau hyperphosphorylation and apoptosis. *In vitro,* certain neurotoxic insults, including exposure to β-amyloid (Aβ), trigger p25 accumulation, Cdk5 activation, and neuronal death, which is averted by pharmacological cdk inhibitors. Moreover, extracellular Aβ deposits induce Cdk5 activation and tau hyperphosphorylation in mutant APP transgenic mice [American Journal of Pathology, 2004, 165, 3, 843-853. Cyclin-Dependent Kinase Inhibitors Attenuate Protein Hyperphosphorylation, Cytoskeletal Lesion Formation, and Motor Defects in Niemann-Pick Type C Mice. Zhang M. et al.; J. Neurosci. Res., 2002, 69, 362-372. p35/ Cdk5 pathway mediates soluble amyloid-beta peptide-induced tau phosphorylation in vitro. *Town T. et al*.].

Cdk5 has a major activity phosphorylating tau in brain [J. Biochem. (Tokyo), 1997, 121, 179- 188. Physiology and pathology of tau protein kinases in relation to Alzheimer's disease. Imahori K. & Uchida T.]. Cdk5, or downstream kinases, can phosphorylate tau prior to, or independently of, aggregation [Neuron, 2003, 38, 555-565. Cdk5 Is a Key Factor in Tau Aggregation and Tangle Formation In Vivo. Noble W. *et al.].* Many Cdk5 phosphorylation sites on tau have been identified. Ser202, Thr205, Thr212, Ser235, Ser396 and Ser404, all Ser/Thr-Pro sites, are the most likely to be physiologically relevant, as several groups have identified them as Cdk5 sites when tau is phosphorylated *in vitro* [Journal of Neurochemistry, 2004, 88, 1313-1326. Cyclin-dependent kinase-5 in neurodegeneration. Shelton S.B. & Johnson G.V.W*;* Neuron, 2003, 38, 555-565. Cdk5 Is a Key Factor in Tau Aggregation and Tangle Formation In Vivo. Noble W. et al.]. Although most of the studies have been carried out *in vitro,* there is some evidence of tau phosphorylation by Cdk5 *in situ* and *in vivo.* Hence, Cdk5 may play a role in the process of NFT formation. Increased Cdk5 activity leads to insoluble aggregation and fibrillization of endogenous tau in the cortex and hippocampus of p25 Tg mice. Furthermore, Cdk5 may indirectly regulate the kinases and phosphatases that act on tau. Cdk5 has been shown to modulate the activity of several protein kinases involved in tau hyperphosphorylation [Neuron, 2003, 40, 471-483. Aberrant Cdk5 Activation by p25 Triggers Pathological Events Leading to Neurodegeneration and Neurofibrillary Tangles. Cruz J.C. et al.; Journal of Neurochemistry, 2004, 88, 1313-1326. Cyclin-dependent kinase-5 in neurodegeneration. Shelton S.B. & Johnson G.V.W].

Additionally, it has been demonstrated that Cdk5 inhibitors can reduce β-amyloid (Aβ) level in the mouse brain [J. Neurosci., 2008, 28, 2624- 2632. Interplay between Cyclin-Dependent Kinase 5 and Glycogen Synthase Kinase 3β Mediated by Neuregulin Signaling Leads to Differential Effects on Tau Phosphorylation and Amyloid Precursor Protein Processing. Wen, Y. *et al*.], since Aβ generation correlates with enhanced Cdk5 activity. Cdk5 has been shown to phosphorylate APP on Thr668 in the cytoplasmic domain. It is reported that T668-phosphorylated APP is up-regulated in AD brain tissues. Moreover, the observations that p25 is elevated in AD brain tissue and in an animal model for β-amyloid and that p25 can be induced by Aβ peptide treatment of primary neurons, indicate that p35 to p25 cleavage may be a downstream event of β-amyloid toxicity [Biochimica et Biophysica Acta 1697 (2004) 137 142. Cdk5, a therapeutic target for Alzheimer's disease?. Tsai L.H., Lee M.S. & Cruz J.].

Cdk5/p25 might also interact with other pathways such as glycogen synthase kinase 3beta (GSK3beta) and c-JUN kinase. Therefore, Cdk5, and other kinases such as glycogen synthase kinase 3β (GSK3β), have been identified as prime candidates for AD pathogenesis because they both generate disease associated phospho-epitopes on tau, and they colocalize with filamentous tau aggregates in the brains of patients and in a transgenic mouse model of tauopathy. Cdk5 and GSK3 also regulate Aβ production *in vivo* [J. Neurosci., 2008, 28, 2624- 2632. Interplay between Cyclin-Dependent Kinase 5 and Glycogen Synthase Kinase 3β Mediated by Neuregulin Signaling Leads to Differential Effects on Tau Phosphorylation and Amyloid Precursor Protein Processing. Wen, Y. *et al*.].

**Pathologies related to Cyclin-dependent kinase 5 (Cdk5)**

Deregulation of cyclin-dependent kinases (Cdks), specifically Cdk5, and cytoskeletal protein hyperphosphorylation characterizes a subset of human neurodegenerative diseases, including Alzheimer's disease [Biochimica et Biophysica Acta 1697 (2004) 137 142. Cdk5, a therapeutic target for Alzheimer's disease?. Tsai L.H., Lee M.S. & Cruz J.], amyotrophic lateral sclerosis [Neurosci. Lett., 1998, 245, 45-48. Cyclin-dependent kinase-5 is associated with lipofuscin in motor neurones in amyotrophic lateral sclerosis. Bajaj *et al*.], Niemann-Pick Type C (NPC) [Neurobiol Dis, 2002, 11, 285-297. Niemann-Pick disease type C yields possible clue for why cerebellar neurons do not form neurofibrillary tangles. Bu B. *et al*.], alcohol-induced neurodegeneration *[*Neurosci. Lett., 2001, 308, 173-176. Ethanol induced changes in cyclin-dependent kinase-5 activity and its activators, P35, P67 (Munc-18) in rat brain Rajgopal Y. & Vemuri M.C.], diffuse Lewy body disease [Am. J. Pathol., 1995, 147, 1465-1476. Cortical and brainstem-type Lewy bodies are immunoreactive for the cyclin-dependent kinase 5. Brion J.P. & Couck A.M.], Parkinson's disease [Proc Natl Acad Sci U S A, 2003, 100, 13650-13655. Cyclin-dependent kinase 5 is a mediator of dopaminergic neuron loss in a mouse model of Parkinson's disease. Smith P.D. *et al.],* progressive supranuclear palsy *[*Neurology, 2002, 58, 589-592. Increase of cdk5 is related to neurofibrillary pathology in progressive supranuclear palsy. Borghi R. *et al.],* frontotemporal dementia and parkinsonism linked to chromosome 17 (FTDP-17) and other tauopathies [J Biol Chem., 2009, 15, 284(20), 13422-33. Familial FTDP-17 missense mutations inhibit microtubule assembly-promoting activity of tau by increasing phosphorylation at Ser202 in vitro. Han D. *et al.],* amnestic mild cognitive impairment [Neurochem Res., 2007, 32(4-5), 655-62.

Regional expression of key cell cycle proteins in brain from subjects with amnestic mild cognitive impairment. Sultana R. & Butterfield D.A.], Down syndrome [Neuroscience, 2008, 22, 156(1), 99-106. Abnormal expression of synaptic proteins and neurotrophin-3 in the Down syndrome mouse model Ts65Dn. Pollonini G. *et al.],* Huntington disease [J Neurosci., 2008, 1, 28(40), 10090-101. Dopaminergic and glutamatergic signaling crosstalk in Huntington's disease neurodegeneration: the role of p25/cyclin-dependent kinase 5. Paoletti P. *et al*.], multiple sclerosis [J Biol Chem., 2004, 31, 279(53), 55833-9. Hyperphosphorylation and aggregation of tau in experimental autoimmune encephalomyelitis. Schneider A. *et al*.], and stroke [Proc Natl Acad Sci USA, 2000, 97, 10254-10259. Cyclin-dependent kinases as a therapeutic target for stroke. Osuga H. *et al*.].

Apart from neurodegenerative diseases, a series of disorders with potential Cdk5 involvement are brain ischemia [Nature Neuroscience, 2003, 6, 1039-1047. Cdk5 activation induces hippocampal CA1 cell death by directly phosphorylating NMDA receptors. Wang J. *et al*.], acute neuronal injury [J Neurochem, 2004, 88(6), 1313-1326. Cyclin-dependent kinase-5 in neurodegeneration. Shelton S.B. & Johnson G.V.W], cocaine addiction [Nature, 2001, 410, 376-380. Effects of chronic exposure to cocaine are regulated by the neuronal protein Cdk5. Bibb J.A. *et al.],* electroconvulsive seizures [J. Neurosci., 2000 20, 8965-8971. Induction of Cyclin-Dependent Kinase 5 in the Hippocampus by Chronic Electroconvulsive Seizures: Role of ΔFosB. Chen J. *et al*.], fetal alcohol syndrome [Neurosci. Lett., 2001, 308, 173-176. Ethanol induced changes in cyclin-dependent kinase-5 activity and its activators, P35, P67 (Munc-18) in rat brain Rajgopal Y. & Vemuri M.C.], focal cortical dysplasia [Dev Neurosci., 2008, 30(1-3), 96-104. The potential role of cyclin-dependent kinase 5 in focal cortical dysplasia. Sen A. *et al*.], hereditary canine spinal muscular atrophy [J. Neuropathol. Exp. Neurol., 1998, 57(11), 991-1099. Alterations in Cyclin-Dependent Protein Kinase 5 (CDK5) Protein Levels, Activity and Immunocytochemistry in Canine Motor Neuron Disease. Green S. *et al*.], inclusion body myositis [Neurology 1999, 53, 1671-1676. Aberrant expression of cyclin-dependent kinase 5 in inclusion body myositis Nakano S. *et al.],* multiple system atrophy [J. Neuropathol. Exp. Neurol., 1998, 57, 690-698. Cyclin-dependent kinase 5 and mitogen-activated protein kinase in glial cytoplasmic inclusions in multiple system atrophy. Nakamura S. *et al*.], peripheral nerve injury *[*Neuroreport., 2002, 13, 243-247. Induction of Cdk5 activity in rat skeletal muscle after nerve injury. Fu W. Y. *et al*.], anxiety and stress [Eur Neuropsychopharmacol., 2010, 20(6), 388-397. Previous stress exposure enhances both anxiety-like behaviour and p35 levels in the basolateral amygdala complex: Modulation by midazolam. Bignante E.A. *et al*.], epilepsy [Synapse, 2009, 63(11), 1017-1028. Gene expression analysis on anterior temporal neocortex of patients with intractable epilepsy. Xi Z. Q. *et al*.], prion diseases [J Neurosci., 2009, 8, 29(27), 8743-8751. Phosphorylation of Prion Protein at Serine 43 Induces Prion Protein Conformational Change. Paresa N. *et al*.], ataxia [Mol Cell Biochem., 2008, 318(1-2), 7-12. Tyrosine hydroxylase expression and Cdk5 kinase activity in ataxic cerebellum. Cheung K.J. *et al*.], schizophrenia [Neuroreport., 2006, 17(18), 1903-5. Expression of p25 impairs contextual learning but not latent inhibition in mice. Mizuno K. *et al*.], pain [Cell Cycle, 2006, 5:6, 585-588. Cdk5: A New Player in Pain Signaling. Tej K. *et al*.], diabetic neuropathy [Rinsho Shinkeigaku., 1999. 39(1), 87-9. New trend in pathogenesis of diabetic neuropathy. Yasuda H.*],* and diabetes mellitus [Mini Rev Med Chem., 2007, 7(10), 1070-4. Cyclin-dependent kinase 5 (Cdk5): a potential therapeutic target for the treatment of neurodegenerative diseases and diabetes mellitus. Wei F. Y. & Tomizawa K.].

***<u>Glycogen synthase kinase-3 (GSK-3)</u>***

Glycogen synthase kinase-3 (GSK-3) is a serine/threonine protein kinase comprised of α and β isoforms that are each encoded by distinct genes (Chemistry & Biology, 2000, 7(10), 793-803. Selective small molecule inhibitors of glycogen synthase kinase-3 modulate glycogen metabolism and gene transcription. Coghlan *et al.;* Curr. Opinion Genetics Dev., 2000, 10(5), 508-514. GSK3, a master switch regulating cell-fate specification and tumorigenesis. Kim, L. & Kimmel, A.R.). GSK-3 plays critical roles in development, metabolic homeostasis, neuronal growth and differentiation, cell polarity, cell fate and in modulation of apoptotic potential.

***Pathologies related to Glycogen synthase kinase-3 (GSK-3)***

Deregulation (usually increase) of GSK-3 activity is believed to play a role in different and important disorders like neurodegenerative disorders [Physiol. Rev., 2004, 84, 361-84. Role of tau protein in both physiological and pathological conditions. Ávila, J. *et al*.], cardiovascular disease [Circ Res., 2009, 104(11), 1240-52; Role of glycogen synthase kinase-3beta in cardioprotection. Juhaszova M. *et al.;* Circ J., 2009, 73(7), 1184-92. GSK-3beta, a therapeutic target for cardiomyocyte protection. Miura T. & Miki T.], diabetes *[*Trends. Mol. Med., 2002, 8, 126-32. Glycogen synthase kinase 3: an emerging therapeutic target. Eldar-Finkelman, H.] or viral infections [Virus Res., 2008, 132, 160-73. Residues in human respiratory syncytial virus P protein that are essential for its activity on RNA viral synthesis. Asenjo, A. *et al*.].

Regarding neurodegenerative disorders and other CNS pathologies, GSK-3 deregulation has been related to Alzheimer's disease [Brain Res Bull., 2009, 80(4-5), 248-50. The role of GSK3 in Alzheimer disease. Hernandez F. *et al*.], Parkinson's disease [Neuroscience Letters 2009, 449(2), 103-107. Glycogen synthase kinase-3beta is associated with Parkinson's disease. Masahiro N. & Hideaki H.], frontotemporal dementia [Arch. Neurol., 2008, 65, 1368-74. Association of GSK3B with Alzheimer disease and frontotemporal dementia.. Schaffer, B. *et al*.], Pick's disease, progressive supranuclear palsy, subacute sclerosing panencephalitis, postencephalitic parkinsonism, dementia pugilistica, guam parkinsonism-dementia complex, corticobasal degeneration, argyrophilic grain disease, familial frontotemporal dementia and parkinsonism linked to chromosome 17 due to mutations in the tau gene (FTDP-17-tau) and other tauopathies [Brain Research Reviews, 2000, 33, 95 -130. Tau protein isoforms, phosphorylation and role in neurodegenerative disorders. Buée, L. *et al*.], AIDS associated dementia [J Neuroimmune Pharmacol., 2007, 2(1), 93-96. Glycogen synthase kinase 3 beta (GSK-3 beta) as a therapeutic target in neuroAIDS. Dewhurst S. *et al*.], Huntington's disease [J Biol Chem., 2002, 277(37), 33791-8. Glycogen synthase kinase-3beta inhibitors prevent cellular polyglutamine toxicity caused by the Huntington's disease mutation. Carmichael J. *et al*.], Lewy body disease *[*Neuropathology., 2003, 23(3), 199-202. Glycogen synthase kinase-3beta phosphorylates synphilin-1 in vitro. Tanji K. *et al.],* bipolar disorder [Neurosci Biobehav Rev., 2007, 31(6), 920-931; GSK-3 is a viable potential target for therapeutic intervention in bipolar disorder. Roew M.K. *et al.;* Bipolar Disord., 2002, 4(2), 137-144. Glycogen Synthase Kinase-3β, mood stabilizers, and neuroprotection. Li X. *et al.],* depression [J Pharmacol Sci., 2009, 110(1), 14-28. Lithium and neuropsychiatric therapeutics: neuroplasticity via glycogen synthase kinase-3beta, beta-catenin, and neurotrophin cascades. Wada A.], schizophrenia [Drug News Perspect., 2007, 20(7), 437-45. The role of glycogen synthase kinase-3beta in schizophrenia. Koros E. & Dorner-Ciossek C.*;* Trends Neurosci., 2007, 30(4), 142-9. Schizophrenia as a GSK-3 dysregulation disorder. Lovestone S. *et al*.], epilepsy [J Neurochem., 1999, 72(3), 1327-30. The mood-stabilizing agent valproate inhibits the activity of glycogen synthase kinase-3. Chen G. *et al*.], mood disorders [Curr Drug Targets., 2006, 7(11), 1421-34. Glycogen synthase kinase-3 (GSK3) in psychiatric diseases and therapeutic interventions. Jope R. S. & Roh M.S.], autism [Proc Natl Acad Sci U S A., 2008, 105(4), 1333-8. Role of GSK3 beta in behavioral abnormalities induced by serotonin deficiency. Beaulieu J.M. *et al*.], attention deficit hyperactivity disorder [Proc Natl Acad Sci U S A., 2004, 101(14), 5099-104. Lithium antagonizes dopamine-dependent behaviors mediated by an AKT/glycogen synthase kinase 3 signaling cascade. Beaulieu J.M. *et al*.], Down's syndrome [FASEB J., 2008, 22(9), 3224-33. Overexpression of Dyrk1A contributes to neurofibrillary degeneration in Down syndrome. Liu F. *et al*.], diseases associated with ischemia/reperfusion and shock *[*Shock., 2007, 27 (2), 113-23. Glycogen synthase kinase 3beta as a target for the therapy of shock and inflammation. Dugo L. *et al*.], brain injury [Neurol Res., 2001, 23(6), 588-92. Different expression of glycogen synthase kinase-3beta between young and old rat brains after transient middle cerebral artery occlusion. Sasaki C. *et al*.], multiple sclerosis [Trends Immunol., 2010, 31(1), 24-31. Innate and adaptive immune responses regulated by glycogen synthase kinase-3 (GSK3). Beurel E. *et al.]* and other autoimmune and inflammatory diseases afflicting the CNS [J. Immunol., 2008, 181(1), 338-45. Lithium prevents and ameliorates experimental autoimmune encephalomyelitis. De Sarno P. *et al*.], spinocerebellar ataxia type 1 [PLoS Med, 2007, 4(5), 836-847. Lithium therapy improves neurological function and hippocampal dendritic arborization in a spinocerebellar ataxia type 1 mouse model. Watase K. *et al.],* cerebral bleeding for example, due to solitary cerebral amyloid angiopathy [Neuroscience., 2008, 153(2), 414-27. Accumulation of beta-amyloid in the brain microvessels accompanies increased hyperphosphorylated tau proteins following microsphere embolism in aged rats. Han F. *et al*.] or amyotrophic lateral sclerosis *[*Brain Res., 2008, 1196, 131-139. Upregulation of GSK3β expression in frontal and temporal cortex in ALS with cognitive impairment (ALSci). Yang W. *et al.].*

In addition to its possible relevance to prevent neurodegeneration, GSK3 inhibitors may also be useful to foster other forms of neuronal repair, including axon regeneration *[*J. Neurosci., 2008, 28, 8914-28. Inactivation of glycogen synthase kinase 3 promotes axonal growth and recovery in the CNS. Dill, J. *et al*.].

During the last few years, GSK-3 has been identified as a regulator of many components of the immune system, suggesting it might be a plausible therapeutic target in inflammatory and autoimmune diseases, such as chronic inflammatory diseases including rheumatoid arthritis, inflammatory bowel disease and psoriasis [Eur J Biochem., 2001, 268 (19), 5001-10. The role of protein phosphorylation in human health and disease. Cohen P.], arthritis [Clin. Immunol., 2006, 120, 57-67. Glycogen synthase kinase-3b inhibition attenuates the degree of arthritis caused by type II collagen in the mouse. Cuzzocrea, S. *et al*.], peritonitis [Immunity, 2006, 24, 563-574. IFN-g suppresses IL-10 production and synergizes with TLR2 by regulating GSK3 and CREBIAP-1 proteins. Hu, X. *et al.],* systemic inflammation, renal dysfunction and hepatotoxicity in endotoxemia [Crit. Care Med., 2005, 33, 1903-1912. GSK-3b inhibitors attenuate the organ injury/dysfunction caused by endotoxemia in the rat. Dugo, L. *et al*.], asthma [Am J Physiol Lung Cell Mol Physiol., 2009, 296(2), L176-84. Airway smooth muscle hyperplasia and hypertrophy correlate with glycogen synthase kinase-3(beta) phosphorylation in a mouse model of asthma. Bentley J.K. *et al*.], sepsis [J. Biochem. Cell. Biol., 2005, 37, 2226-2238. GSK-3b inhibitors reduce protein degradation in muscles from septic rats and in dexamethasone treated myotubes. Int. Evenson, A.R. *et al*.], colitis [Br. J. Pharmacol., 2006, 147, 575-582. Reduction of experimental colitis in the rat by inhibitors of glycogen synthase kinase-3b. Whittle, B.J. *et al.],* inflammation-induced organ injury caused by hemorrhage and resuscitation [Shock, 2006, 25, 485-491. Glycogen synthase kinase-3b inhibitors protect against the organ injury and dysfunction caused by hemorrhage and resuscitation. Dugo, L. *et al.],* inflammatory injury in chronic renal allograft disease [Am J Transplant., 2008, 8(9), 1852-63. Glycogen synthase kinase 3beta: a novel marker and modulator of inflammatory injury in chronic renal allograft disease. Gong R. *et al*.] or lupus [Int. J. Immunopharmacol., 1995, 17, 581-592. Lithium chloride enhances survival of NZB/W lupus mice: influence of melatonin and timing of treatment. Lenz,

S.P. *et al*.].

Among cardiovascular disorders related to GSK-3 are heart disease [Circ. Res., 2002, 90, 1055-63. Glycogen synthase kinase-3beta: a novel regulator of cardiac hypertrophy and development. Hardt, S.E. & Sadoshima, J.], atherosclerosis [Am J Pathol., 2009, 174(1), 330-42. Valproate attenuates accelerated atherosclerosis in hyperglycemic apoE-deficient mice: evidence in support of a role for endoplasmic reticulum stress and glycogen synthase kinase-3 in lesion development and hepatic steatosis. Bowes A.J. *et al*.], hypertension [J. Clin. Invest., 2002, 109(3), 373-381. Fas receptor signaling inhibits glycogen synthase kinase 3β and induces cardiac hypertrophy following pressure overload. Badorff C. *et al*.], restenosis [Cardiovasc Res., 2010, Epub. Delayed Re-endothelialization with Rapamycin-coated Stents is Rescued by the Addition of a Glycogen Synthase Kinase 3 Beta Inhibitor. Ma X. *et al.*] or leukopenia [Gallicchio, V. S. (1991) in Lithium and the Cell, ed. Birch, N. J. (Academic, San Diego), pp. 185-198.].

Additional pathologies associated with GSK-3 are metabolic syndrome X [Curr Pharm Des., 2004, 10(10), 1105-37. Discovery and development of GSK3 inhibitors for the treatment of type 2 diabetes. Wagman A.S. *et al*.], hair loss [J Clin Invest., 2010, 120(2), 446-56. Neural Wiskott-Aldrich syndrome protein modulates Wnt signaling and is required for hair follicle cycling in mice. Lyubimova A. *et al*.], severe acute respiratory syndrome coronavirus [J Biol Chem., 2009, 284(8), 5229-39. Glycogen synthase kinase-3 regulates the phosphorylation of severe acute respiratory syndrome coronavirus nucleocapsid protein and viral replication. Wu C.H. *et al*.], cocaine addiction [J Neurochem., 2009, 111(6), 1357-68. Glycogen synthase kinase 3beta in the nucleus accumbens core mediates cocaine-induced behavioral sensitization. Xu C.M. *et al*.], bone loss [Life Sci., 2009, 85(19-20)y 685-92. Inhibition of glycogen synthase kinase-3beta attenuates glucocorticoid-induced bone loss. Wang F.S. *et al.],* fragile X syndrome (FXS) [Biochem Pharmacol., 2010, 79(4), 632-46. Lithium ameliorates altered glycogen synthase kinase-3 and behavior in a mouse model of fragile X syndrome. Yuskaitis C.J. *et al*.] or glaucoma [J Clin Invest., 2008, 118(3), 1056-64. Increased expression of the WNT antagonist sFRP-1 in glaucoma elevates intraocular pressure. Wang WH. *et al*.].

### *GSK-3 inhibitors*

For a further review of GSK-3 inhibitors and their use as potential treatments for these pathologies, please reference to Nature Reviews, 2004, 3, 479-487. GSK3 inhibitors: development end therapeutic potential. Cohen, P. & Goedert, M.*;* Mini-Reviews in Medicinal Chemistry, 2009, 9(9), 1024-1029. GSK3 Inhibitors and Disease. Hernández, F. *et al.;* Curr. Opin. Drug Discov. Develop., 2008, 11(4), 533-543. Glycogen synthase kinase-3 (GSK-3) inhibitors reach the clinic. Medina, M. & Castro, A.*;* John Wiley & Sons, Inc., 2006. Glycogen Synthase Kinase 3 (GSK-3) and its inhibitors. Chapter 14. Eds: Martinez, A., Castro, A. & Medina, M.

Several GSK-3 inhibitors like indirubines [J. Biol. Chem., 2001, 276, 251-60. Indirubins inhibit glycogen synthase kinase-3 beta and CDK5/p25, two protein kinases involved in abnormal tau phosphorylation in Alzheimer's disease. A property common to most cyclin-dependent kinase inhibitors?. Leclerc, S. *et al*.], maleimides [Bioorg. Med. Chem. Lett., 2001, 11, 635-9. 3-Anilino- 4-arylmaleimides: potent and selective inhibitors of glycogen synthase kinase-3 (GSK-3). Smith, D. *et al*.], 3-amino pyrazoles *[*Bioorg. Med. Chem. Lett., 2003, 13, 1581-4. 5-arylpyrazolo[3,4-b]pyridazines: potent inhibitors of glycogen synthase kinase-3 (GSK-3). Witherington, J. *et al.],* paullones [Eur. J. Biochem., 2000, 267, 5983-94. Paullones are potent inhibitors of glycogen synthase kinase-3beta and cyclin-dependent kinase 5/p25. Leost, M. *et al*.], thiazoles [J. Biol. Chem., 2003, 278, 45937-45. Structural insights and biological effects of glycogen synthase kinase 3-specific inhibitor AR-A014418. Bhat, R. *et al.]* or thiadiazolidinones [J. Med. Chem., 2002, 45, 1292-9. First non-ATP competitive glycogen synthase kinase 3 beta (GSK-3beta) inhibitors: thiadiazolidinones (TDZD) as potential drugs for the treatment of Alzheimer's disease. Martinez, A. *et al*.] have been described.

In view of the above, it is of great potential therapeutic interest to find further GSK-3 inhibitors, which may be useful for treating several diseases or conditions as detailed above.

### Indole-dihydro-imidazole derivatives

Some indole-dihydro-imidazole derivatives have been described in the literature, for example in Org. Biomol. Chem., 2008, 6, 2765-2771, wherein some particular compounds have been described having cytotoxic properties.

## BRIEF DESCRIPTION OF THE INVENTION

It has been found that indole-dihydro-imidazole derivatives of Formula (I):

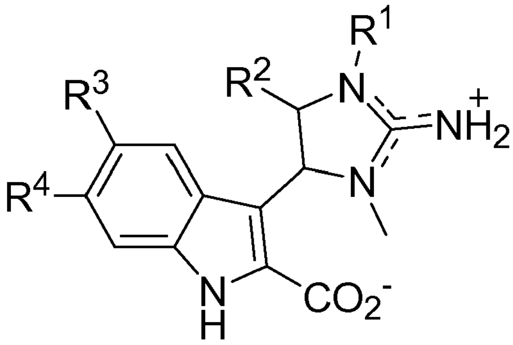

surprisingly inhibit Cdk5 and GSK3 activity, thus having a potential therapeutic interest for the treatment of diseases or conditions wherein Cdk5 and GSK3 are involved and/or a modulation thereof is necessary.

$R^1$, $R^2$, $R^3$ and $R^4$ in Formula I have the following meanings:

$R^1$ is selected from methyl and hydrogen or is absent, depending on the position of the double bond in the imidazole ring;
$R^2$ is selected from hidroxy and hydrogen;
$R^3$ is selected from bromo and hydrogen;
$R^4$ is selected from hidroxy and hydrogen.

Therefore, according to a first aspect, the present invention is directed to a compound of Formula (I), as defined above, or a tautomer, salt, solvate or prodrug thereof, for its use in the treatment of a disease or condition mediated by Cdk5 or GSK3.

According to a further aspect, the present invention is directed to the use of a compound of Formula (I), as defined above, or a tautomer, salt, solvate or prodrug thereof, for the manufacture of a medicament for the treatment of a disease or condition mediated by Cdk5 or GSK3.

According to another aspect, the present invention is directed to a method of treatment of a disease or condition mediated by Cdk5 or GSK3, said method comprising administering a compound of Formula (I), as defined above, or a tautomer, salt, solvate or prodrug thereof, to a patient in need of treatment.

Finally, another aspect of the present invention relates to the use of a compound of formula (I), or a tautomer, salt, solvate or prodrug thereof, as defined above, as a reactive for a biological assay.

## DETAILED DESCRIPTION OF THE INVENTION

The disease or condition mediated by Cdk5 or GSK3 in the frame of the present invention is preferably selected from neurodegenerative disorders, CNS pathologies, inflammatory and autoimmune diseases, cardiovascular diseases, diabetes mellitus and viral infections.

Particularly, the neurodegenerative disorder or CNS pathology is preferably, but not limited to, selected from Alzheimer's disease, Parkinson's disease, tauopathies, frontotemporal dementia, Pick's disease, Niemann-Pick Type C (NPC), progressive supranuclear palsy, subacute sclerosing panencephalitis, postencephalitic parkinsonism, dementia pugilistica, guam parkinsonism-dementia complex, corticobasal degeneration, argyrophilic grain disease, familial frontotemporal dementia and parkinsonism linked to chromosome 17 due to mutations in the tau gene (FTDP-17-tau), AIDS associated dementia, Huntington's disease, Lewy body disease, bipolar disorder, depression, schizophrenia, epilepsy, mood disorders, autism, attention deficit hyperactivity disorder, Down's syndrome, brain ischemia and diseases associated with ischemia/reperfusion and shock, brain injury, multiple sclerosis and other autoimmune and inflammatory diseases afflicting the CNS, spinocerebellar ataxia type 1, cerebral bleeding, for example due to solitary cerebral amyloid angiopathy, amyotrophic lateral sclerosis, alcohol-induced neurodegeneration, amnestic mild cognitive impairment, stroke, fragile X syndrome (FXS), acute neuronal injury, electroconvulsive seizures, focal cortical dysplasia, hereditary canine spinal muscular atrophy, multiple system atrophy, anxiety and stress, prion diseases, ataxia or pain.

The inflammatory and autoimmune disease is preferably selected from chronic inflammatory diseases including rheumatoid arthritis, inflammatory bowel disease and psoriasis, arthritis, peritonitis, systemic inflammation, renal dysfunction and hepatotoxicity in endotoxemia, asthma, sepsis, colitis, inflammation-induced organ injury caused by hemorrhage and resuscitation, inflammatory injury in chronic renal allograft disease or lupus.

The cardiovascular disorder is preferably selected from heart disease, brain ischemia, atherosclerosis, hypertension, restenosis or leukopenia.

The Cdk5 and GSK3 mediated disease may also be selected from additional pathologies, such as metabolic syndrome X, hair loss, severe acute respiratory syndrome coronavirus, cocaine addiction, bone loss, glaucoma, fetal alcohol syndrome, inclusion body myositis, peripheral nerve injury and diabetic neuropathy.

Therefore, the Cdk5 and GSK3 mediated disease may be selected from Alzheimer's disease, Parkinson's disease, tauopathies, frontotemporal dementia, Pick's disease, Niemann-Pick Type C (NPC), progressive supranuclear palsy, subacute sclerosing panencephalitis, postencephalitic parkinsonism, dementia pugilistica, guam parkinsonism-dementia complex, corticobasal degeneration, argyrophilic grain disease, familial frontotemporal dementia and parkinsonism linked to chromosome 17 due to mutations in the tau gene (FTDP-17-tau), AIDS associated dementia, Huntington's disease, Lewy body disease, bipolar disorder, depression, schizophrenia, epilepsy, mood disorders, autism, attention deficit hyperactivity disorder, Down's syndrome, diseases associated with ischemia/reperfusion and shock, brain injury, multiple sclerosis and other autoimmune and inflammatory diseases afflicting the CNS, spinocerebellar ataxia type 1, cerebral bleeding, for example due to solitary cerebral amyloid angiopathy, amyotrophic lateral sclerosis, alcohol-induced neurodegeneration, amnestic mild cognitive impairment, stroke, chronic inflammatory diseases including rheumatoid arthritis, inflammatory bowel disease and psoriasis, arthritis, peritonitis, systemic inflammation, renal dysfunction and hepatotoxicity in endotoxemia, asthma, sepsis, colitis, inflammation-induced organ injury caused by hemorrhage and resuscitation, inflammatory injury in chronic renal allograft disease, lupus, heart disease, brain ischemia, atherosclerosis, hypertension, restenosis or leukopenia, metabolic syndrome X, hair loss, severe acute respiratory syndrome coronavirus, cocaine addiction, bone loss, fragile X syndrome (FXS), glaucoma, acute neuronal injury, electroconvulsive seizures, fetal alcohol syndrome, focal cortical dysplasia, hereditary canine spinal muscular atrophy, inclusion body myositis, multiple system atrophy, peripheral nerve injury, anxiety and stress, prion diseases, ataxia, pain and diabetic neuropathy.

The compounds of Formula I may be in the form of tautomers, in the form of salts, preferably pharmaceutically acceptable salts, in the form of solvates or in the form of prodrugs.

The term "pharmaceutically acceptable salts" refers to salts which, upon administration to the recipient are capable of providing (directly or indirectly) a compound as described herein. The preparation of salts can be carried out by methods known in the art. Preferably, "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

For instance, pharmaceutically acceptable salts of compounds provided herein are synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, nonaqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulfonate and p-toluenesulfonate. Examples of the alkali addition salts include inorganic salts such as, for example, sodium, potassium, calcium, ammonium, magnesium, aluminium and lithium salts, and organic alkali salts such as, for example, ethylenediamine, ethanolamine, N,N-dialkylenethanolamine, triethanolamine, glucamine and basic aminoacids salts.

The term "prodrug" as used in this application is defined here as meaning a chemical compound having undergone a chemical derivation such as substitution or addition of a further chemical group to change (for pharmaceutical use) any of its physico-chemical properties, such as solubility or bioavailability, e.g. ester and ether derivatives of an active compound that yield the active compound per se after administration to a subject. Examples of well known methods of producing a prodrug of a given acting compound are known to those skilled in the art and can be found e.g. in Krogsgaard-Larsen et al., Textbook of Drug design and Discovery, Taylor & Francis (April 2002).

Particularly favoured prodrugs are those that increase the bioavailability of the compounds of this invention when such compounds are administered to a patient (e.g., by allowing an orally administered compound to be more readily absorbed into the blood) or which enhance delivery of the parent compound to a biological compartment (e.g., the brain or lymphatic system) relative to the parent species.

The term "solvate" according to this invention is to be understood as meaning any form of the active compound according to the invention which has another molecule (most likely a polar solvent) attached to it via a non-covalent bonding. Examples of such solvates include hydrates and alcoholates, e.g. methanolates.

The preparation of salts, solvates and prodrugs can be carried out by methods known in the art. It will be appreciated that non-pharmaceutically acceptable salts, solvates or prodrugs also fall within the scope of the invention since those may be useful in the preparation of pharmaceutically acceptable salts, solvates or prodrugs.

The compounds of the present invention may exhibit tautomerism. Tautomers are one of two or more structural

isomers of a compound that exist in equilibrium and are readily converted from one isomeric form to another. Common tautomeric pairs are amine-imine, amide-imidic acid, keto-enol, lactam-lactim, etc. For example, the compounds of the present invention may be equally represented as:

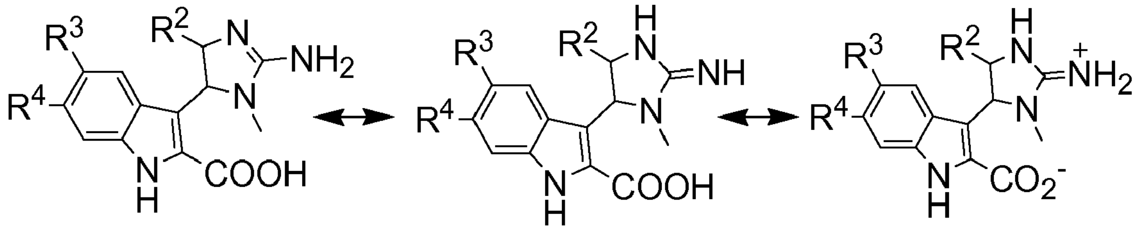

Generally a "therapeutically effective amount" of the compound of the invention or a pharmaceutical composition thereof will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000 mg/kg/day.

The term "treatment" or "to treat" in the context of this specification means administration of a compound or formulation according to the invention to prevent, ameliorate or eliminate the disease or one or more symptoms associated with said disease. "Treatment" also encompasses preventing, ameliorating or eliminating the physiological sequelae of the disease.

The term "ameliorate" in the context of this invention is understood as meaning any improvement on the situation of the patient treated - either subjectively (feeling of or on the patient) or objectively (measured parameters).

Preferably, the compounds of Formula I are selected from trachycladindoles A-G, for which in Formula (I) $R^1$ to $R^4$ have the following meanings:

| Trachycladindole | $R^3$ | $R^4$ | $R^2$ | $R^1$ |
|---|---|---|---|---|
| **A** | Br | H | H | H |
| **B** | Br | H | H | Me |
| **C** | Br | OH | H | H |
| **D** | Br | OH | H | Me |
| **E** | Br | H | OH | Me |
| **F** | Br | OH | OH | Me |
| **G** | H | H | H | H |

or a tautomer, salt, solvate or prodrug thereof.

An additional aspect of the present invention relates to the use of a compound of formula (I), or a tautomer, salt, solvate or prodrug thereof, as defined above, as a reactive for a biological assay. In a preferred embodiment, the biological assay consists in the Cdk5 or GSK3 inhibition. More preferably, the biological assay is an in vitro assay.

## <u>EXAMPLES</u>

### OBTENTION AND IDENTIFICATION

The isolation, characterization and structure elucidation of trachycladindoles A-G were described for the first time by Capon R.J., Peng C. & Dooms C. (Trachycladindoles A-G: cytotoxic heterocycles from an Australian marine sponge, Trachycladus laevispirulifer. Organic & Biomolecular Chemistry, 2008, 6, 2765-2771). This document is herewith incorporated by reference into the present application.

### BIOLOGICAL DATA

### Example 1: Cdk5 assay

The enzymatic activity of Cdk5 was determined with a commercial system based on the Z'-LYTE® technology,

available from Life Technologies (Carlsbad, CA, USA), using human recombinant Cdk5/p35 heterodimer, also available from Invitrogen as the enzyme source. This technology utilizes the fluorescence resonance energy transfer ("FRET") process between fluorescein and coumarin. The assay principle is based on the differential sensitivity of phosphorylated and non-phosphorylated peptide to proteolytic cleavage, which precludes the energy transfer process between the two fluorophores attached to both sides of the cleavage site. Hence, phosphorylation by Cdk5/p35 will yield a phosphopeptide which cannot be hydrolyzed by a suitable protease and energy transfer between the two fluorophores will occur. Opposingly, lack of phosphorylation will cause peptide hydrolysis hence lack of energy transfer. The assay is performed in 384-well black plates, in a final volume of 10 μl, with 10 nM enzyme concentration, in 50 mM Hepes pH 7.5, 50 mM MgCl2, 5 mM EGTA and 0.05% Brij-35, using 12.5 μM ATP and 2 μM substrate peptide. The latter is a synthetic peptide provided by Invitrogen, under the commercial name "Ser/Thr 12 peptide", and it is based upon the sequence of Cdk7, containing Ser-164 and a (Ser/Thr)-Pro-X-(Lys/Arg) consensus sequence, wherein X is any residue. The peptide is labeled at both ends with fluorescein and coumarin. The assay is carried out in the presence of different concentrations of the tested compound, at a final 1% (v/v) DMSO concentration. After a 60 min incubation, at room temperature, 5 μl of a commercial protease solution (sold by the same vendor in the assay kit) is added and a subsequent 1h incubation at room temperature is performed, before adding 5 μl of a suitable "stop solution", also provided by the vendor in the kit. After that, fluorescence intensity is recorded, monitoring emission at both 445 and 520 nm, upon excitation at 400 nm. An emission ratio is finally calculated, using the quotient among the emission at 445 nm divided by that at 520 nm.

In the assay plate, several wells are included as a control for full enzyme activity, these wells do not contain any inhibitor or tested substance. Likewise, several wells are also included as a control for lack of enzyme activity, thus these wells do not contain inhibitor nor enzyme. The emission ratio of each tested sample is normalized to that of the control wells, so that for every compound concentration the percentage of inhibition is calculated by using the following equation:

$$\% \text{ Inhibition} = 100 \cdot \frac{E-S}{E-B}$$

where "S" is the emission from the wells with the tested sample, "E" is the average emission from control wells with full enzyme activity and "B" is the average emission from wells with full enzyme inhibition. Inhibition values obtained for every compound concentration are finally used to calculate the pEC50 of the tested compound, this parameter being the negative value of the logarithm of the compound concentration, in M units, causing 50% of its maximum effect (i.e., the higher the pEC50 value the higher the potency of the compound). For that purpose the data were fitted to the following equation using the nonlinear regression function of GraphPad™ Prism 5.0 (GraphPad Software Inc.):

$$\% \text{ Inhibition} = L + \frac{H-L}{1+10^{(\log C + pEC50)^{N}}}$$

where "L" is the lower asymptote of the theoretical sigmoidal curve, "H" is the higher asymptote, "C" is the concentration of compound in M units and "N" is the Hill coefficient.

In **Table 1**, the pEC50 values obtained for some compounds of Formula I are indicated:

**Table 1**

| Compound No | Structure | Cdk5 inhibition pEC50 average |
|---|---|---|
| Compound 1 | 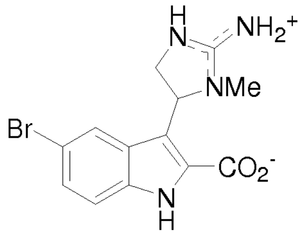 | 5.96 |
| Compound 2 | 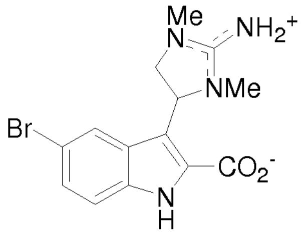 | 5.55 |
| Compound 3 | 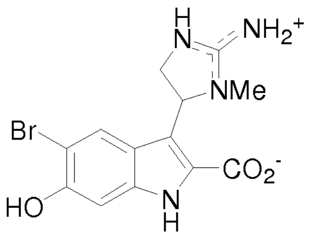 | 7.02 |
| Compound 4 | 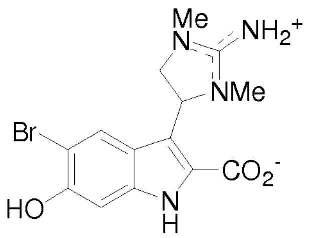 | 7.13 |
| Compound 5 | 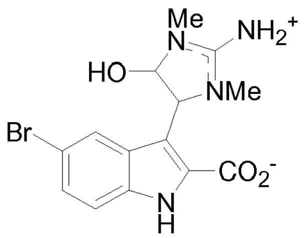 | 5.51 |
| Compound 6 | 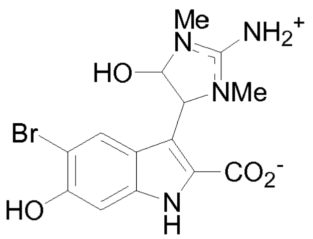 | 7.31 |

(continued)

| Compound No | Structure | Cdk5 inhibition |
|---|---|---|
| | | pEC50 average |
| Compound 7 | 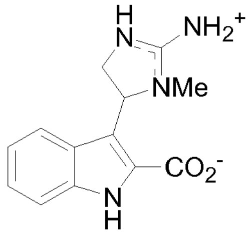 | 6.03 |

**Example 2: GSK-3 assay**

The enzymatic activity of GSK3β was determined with a commercial system based on the Z'-LYTE® technology, available from Life Technologies (Carlsbad, CA, USA), using human recombinant GSK3β (N-terminal 6His-tagged recombinant enzyme with an H350L mutation) from Millipore (Billerica, MA, USA) as the enzyme source. This technology utilizes the fluorescence resonance energy transfer ("FRET") process between fluorescein and coumarin. The assay principle is based on the differential sensitivity of phosphorylated and non-phosphorylated peptide to proteolytic cleavage, which precludes the energy transfer process between the two fluorophores attached to both sides of the cleavage site. Hence, phosphorylation by GSK3β will yield a phosphopeptide, which cannot be hydrolyzed by a suitable protease and energy transfer between the two fluorophores will occur. Opposingly, lack of phosphorylation will cause peptide hydrolysis hence lack of energy transfer. The assay is performed in 384-well black plates, in a final volume of 10 μl, with 2 nM enzyme concentration in 50 mM Hepes pH 7.5, 50 mM $MgCl_2$, 5 mM EGTA and 0.05% Brij-35, using 12.5 μM ATP and 2 μM substrate peptide. The latter is a synthetic peptide, provided by Invitrogen under the commercial name "Ser/Thr 9 peptide", and it is based upon the sequence of a GSK3β substrate protein (glycogen synthase I) containing Ser-641. The peptide is labeled at both ends with fluorescein and coumarin. The assay is carried out in the presence of different concentrations of the tested compound, at a final 1 % (v/v) DMSO concentration. After a 60 min incubation at room temperature, 5 μl of a commercial protease solution (sold by the same vendor in the assay kit) is added and a subsequent 1h incubation at room temperature is performed, before adding 5 μl of a suitable "stop solution", also provided by the vendor in the kit. After that, fluorescence intensity is recorded, monitoring emission at both 445 and 520 nm, upon excitation at 400 nm. An emission ratio is finally calculated, using the quotient among the emission at 445 nm divided by that at 520 nm.

The emission ratios for each concentration of compound are finally used to calculate the pEC50 value, according to the procedure described above for Cdk5.

In **Table 2,** the pEC50 values obtained for some compounds of Formula I are indicated:

<u>**Table 2**</u>

| Compound No | Structure | GSK-3 inhibition |
|---|---|---|
| | | pEC50 average |
| Compound 1 | See table 1 | 4.45 |
| Compound 2 | See table 1 | 4.13 |
| Compound 3 | See table 1 | 4.95 |
| Compound 4 | See table 1 | 4.81 |
| Compound 5 | See table 1 | 4.01 |
| Compound 6 | See table 1 | 5.08 |
| Compound 7 | See table 1 | 4.37 |

## Claims

**1.** A compound of Formula (I):

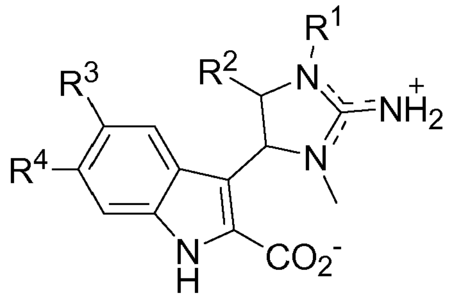

wherein
$R^1$ is selected from methyl and hydrogen;
$R^2$ is selected from hidroxy and hydrogen;
$R^3$ is selected from bromo and hydrogen;
$R^4$ is selected from hidroxy and hydrogen;
or a tautomer, salt, solvate or prodrug thereof, for its use in the treatment of a disease or condition mediated by Cdk5 or GSK3.

**2.** The compound according to claim 1, wherein the disease or condition is selected from neurodegenerative disorders, CNS pathologies, inflammatory and autoimmune diseases, cardiovascular diseases, diabetes mellitus and viral infections.

**3.** The compound according to claim 1, wherein the disease or condition is selected from Alzheimer's disease, Parkinson's disease, tauopathies, frontotemporal dementia, Pick's disease, Niemann-Pick Type C (NPC), progressive supranuclear palsy, subacute sclerosing panencephalitis, postencephalitic parkinsonism, dementia pugilistica, guam parkinsonism-dementia complex, corticobasal degeneration, argyrophilic grain disease, familial frontotemporal dementia and parkinsonism linked to chromosome 17 due to mutations in the tau gene (FTDP-17-tau), AIDS associated dementia, Huntington's disease, Lewy body disease, bipolar disorder, depression, schizophrenia, epilepsy, mood disorders, autism, attention deficit hyperactivity disorder, Down's syndrome, diseases associated with ischemia/ reperfusion and shock, brain injury, multiple sclerosis and other autoimmune and inflammatory diseases afflicting the CNS, spinocerebellar ataxia type 1, cerebral bleeding, for example due to solitary cerebral amyloid angiopathy, amyotrophic lateral sclerosis, alcohol-induced neurodegeneration, amnestic mild cognitive impairment, stroke, chronic inflammatory diseases including rheumatoid arthritis, inflammatory bowel disease and psoriasis, arthritis, peritonitis, systemic inflammation, renal dysfunction and hepatotoxicity in endotoxemia, asthma, sepsis, colitis, inflammation-induced organ injury caused by hemorrhage and resuscitation, inflammatory injury in chronic renal allograft disease, lupus, heart disease, brain ischemia, atherosclerosis, hypertension, restenosis or leukopenia, metabolic syndrome X, hair loss, severe acute respiratory syndrome coronavirus, cocaine addiction, bone loss, fragile X syndrome (FXS), glaucoma, acute neuronal injury, electroconvulsive seizures, fetal alcohol syndrome, focal cortical dysplasia, hereditary canine spinal muscular atrophy, inclusion body myositis, multiple system atrophy, peripheral nerve injury, anxiety and stress, prion diseases, ataxia, pain and diabetic neuropathy.

**4.** The compound according to any one of claims 1 to 3, wherein the compound of Formula (I) is selected from:

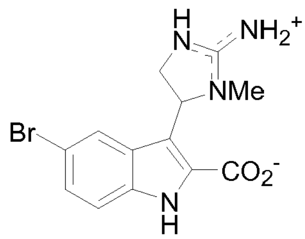

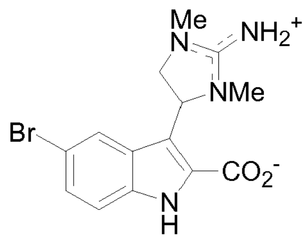

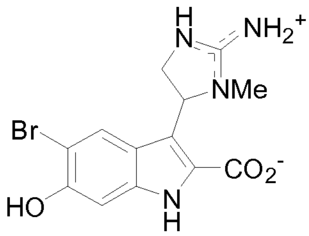

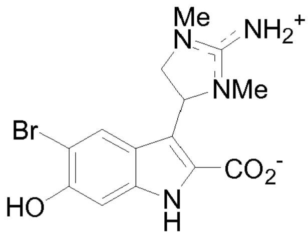

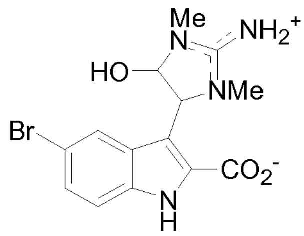

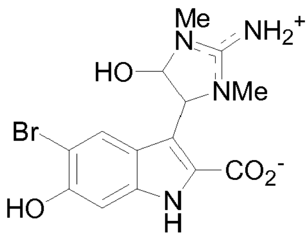

and

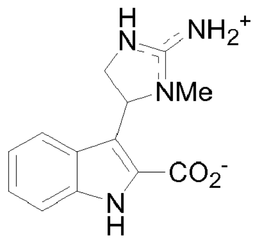

or a tautomer, salt, solvate or prodrug thereof.

**5.** Use of a compound of Formula (I):

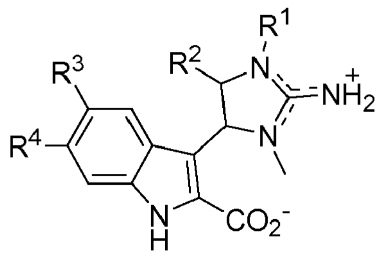

wherein
$R^1$ is selected from methyl and hydrogen;
$R^2$ is selected from hidroxy and hydrogen;
$R^3$ is selected from bromo and hydrogen;
$R^4$ is selected from hidroxy and hydrogen;
or a tautomer, salt, solvate or prodrug thereof, for the manufacture of a medicament for the treatment of a disease or condition mediated by Cdk5 or GSK3.

**6.** The use according to claim 5, wherein the disease or condition is selected from neurodegenerative disorders, CNS pathologies, inflammatory and autoimmune diseases, cardiovascular diseases, diabetes mellitus and viral infections.

**7.** The use according to claim 5, wherein the disease or condition is selected from Alzheimer's disease, Parkinson's disease, tauopathies, frontotemporal dementia, Pick's disease, Niemann-Pick Type C (NPC), progressive supranuclear palsy, subacute sclerosing panencephalitis, postencephalitic parkinsonism, dementia pugilistica, guam parkinsonism-dementia complex, corticobasal degeneration, argyrophilic grain disease, familial frontotemporal dementia and parkinsonism linked to chromosome 17 due to mutations in the tau gene (FTDP-17-tau), AIDS associated dementia, Huntington's disease, Lewy body disease, bipolar disorder, depression, schizophrenia, epilepsy, mood disorders, autism, attention deficit hyperactivity disorder, Down's syndrome, diseases associated with ischemia/ reperfusion and shock, brain injury, multiple sclerosis and other autoimmune and inflammatory diseases afflicting the CNS, spinocerebellar ataxia type 1, cerebral bleeding, for example due to solitary cerebral amyloid angiopathy, amyotrophic lateral sclerosis, alcohol-induced neurodegeneration, amnestic mild cognitive impairment, stroke, chronic inflammatory diseases including rheumatoid arthritis, inflammatory bowel disease and psoriasis, peritonitis, arthritis, systemic inflammation, renal dysfunction and hepatotoxicity in endotoxemia, asthma, sepsis, colitis, inflammation-induced organ injury caused by hemorrhage and resuscitation, inflammatory injury in chronic renal allograft disease, lupus, heart disease, brain ischemia, atherosclerosis, hypertension, restenosis or leukopenia, metabolic syndrome X, hair loss, severe acute respiratory syndrome coronavirus, cocaine addiction, bone loss, fragile X syndrome (FXS), glaucoma, acute neuronal injury, electroconvulsive seizures, fetal alcohol syndrome, focal cortical dysplasia, hereditary canine spinal muscular atrophy, inclusion body myositis, multiple system atrophy, peripheral nerve injury, anxiety and stress, prion diseases, ataxia, pain and diabetic neuropathy.

**8.** The use according to any one of claims 5 to 7, wherein the compound of Formula (I) is selected from:

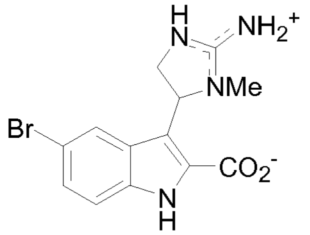
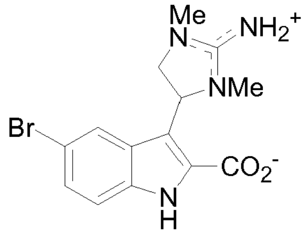

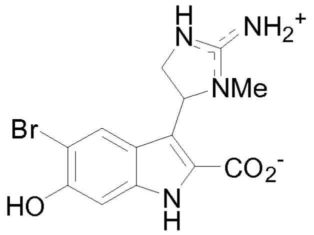
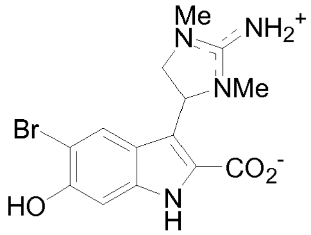

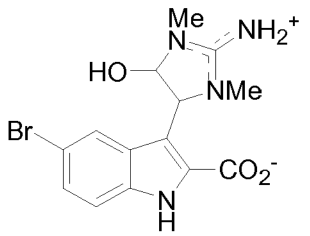
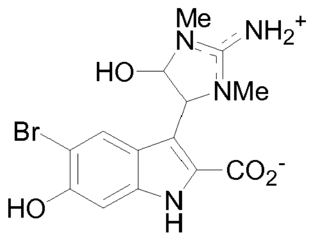

and

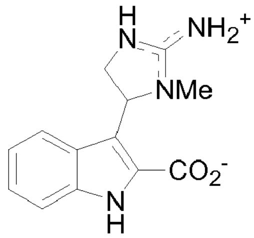

or a tautomer, salt, solvate or prodrug thereof,

**9.** Use of a compound of formula (I), or a tautomer, salt, solvate or prodrug thereof, as defined in any one of claims 1, 4, 5 or 8, as a reactive for a biological assay.

**10.** The use according to claim 9, wherein the biological assay consists in the Cdk5 or GSK3 inhibition.

**11.** The use according to any one of claims 9 and 10, wherein the biological assay is an *in vitro* assay.

Europäisches Patentamt
European Patent Office
Office européen des brevets

# EUROPEAN SEARCH REPORT

Application Number
EP 12 16 7268

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | DATABASE MEDLINE [Online] US NATIONAL LIBRARY OF MEDICINE (NLM), BETHESDA, MD, US; 7 August 2008 (2008-08-07), CAPON ROBERT J ET AL: "Trachycladindoles A-G: cytotoxic heterocycles from an Australian marine sponge, Trachycladus laevispirulifer.", XP002683302, Database accession no. NLM18633534 * abstract * & CAPON ROBERT J ET AL: "Trachycladindoles A-G: cytotoxic heterocycles from an Australian marine sponge, Trachycladus laevispirulifer.", ORGANIC & BIOMOLECULAR CHEMISTRY 7 AUG 2008 LNKD- PUBMED:18633534, vol. 6, no. 15, 7 August 2008 (2008-08-07), pages 2765-2771, ISSN: 1477-0520 ----- | 5-10 | INV. A61K31/4178 A61P9/00 A61P3/10 A61P25/00 A61P29/00 A61P31/12 A61P37/02 |
| X | CN 101 260 102 B (UNIV SHANGHAI JIAOTONG [CN] UNIV SHANGHAI JIAOTONG) 10 September 2008 (2008-09-10) * abstract * * the whole document * ----- | 5-10 | TECHNICAL FIELDS SEARCHED (IPC) A61K |

The present search report has been drawn up for all claims

| Place of search | Date of completion of the search | Examiner |
|---|---|---|
| Munich | 14 September 2012 | Collura, Alessandra |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

**ANNEX TO THE EUROPEAN SEARCH REPORT ON EUROPEAN PATENT APPLICATION NO.** EP 12 16 7268

This annex lists the patent family members relating to the patent documents cited in the above-mentioned European search report.
The members are as contained in the European Patent Office EDP file on
The European Patent Office is in no way liable for these particulars which are merely given for the purpose of information.

14-09-2012

| Patent document cited in search report | Publication date | Patent family member(s) | Publication date |
|---|---|---|---|
| CN 101260102 B | 10-09-2008 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- A decade of CDK5. *Nat. Rev., Mol. Cell Biol.,* 2001, vol. 2, 749-759
- **DHAVAN R. ; TSAI L.H.** Cyclin-dependent kinase-5 in neurodegeneration. *Journal of Neurochemistry,* 2004, vol. 88, 1313-1326
- **SHELTON S.B. ; JOHNSON G.V.W.** An Unusual Member of the Cdk Family: Cdk5. *Cell Mol Neurobiol,* 2008, vol. 28, 351-369
- **DHAVAN R. ; TSAI L.H.** A decade of CDK5. *Nat. Rev., Mol. Cell Biol.,* 2001, vol. 2, 749-759
- Cdk5, a therapeutic target for Alzheimer's disease?. *Biochimica et Biophysica Acta,* 2004, vol. 1697, 137-142
- **TSAI L.H. ; LEE M.S. ; CRUZ J.** An Unusual Member of the Cdk Family: Cdk5. Dhariwala F.A. & Rajadhyaksha M.S. *Cell Mol Neurobiol,* 2008, vol. 28, 351-369
- Callosal axon guidance defects in p35 (-/-) mice. *J. Comp. Neurol.,* 1999, vol. 415, 218-229
- **KWON Y. T., ; TSAI L.H. ; CRANDALL J. E.** Cyclin-dependent kinase-5 in neurodegeneration. *Journal of Neurochemistry,* 2004, vol. 88, 1313-1326
- **SHELTON S.B. ; JOHNSON G.V.W.** Cyclin-dependent kinase-5 in neurodegeneration. *Journal of Neurochemistry,* 2004, vol. 88, 1313-1326
- **TSAI L.H. ; LEE M. S. ; CRUZ J.** Cdk5, a therapeutic target for Alzheimer's disease?. *Biochimica et Biophysica Acta,* 2004, vol. 1697, 137-142
- **TSAI L. H.** Activity and expression pattern of cyclin-dependent kinase 5 in the embryonic mouse nervous system. *Development,* 2003, vol. 119, 1029-1040
- A brain-specific activator of cyclin-dependent kinase 5. *Nature,* 1994, vol. 371, 423-426
- **LEW et al.** p35 is a neural-specific regulatory subunit of cyclin-dependent kinase 5. *Nature,* 1994, vol. 371, 419-423
- **TSAI et al.** Precursor of cdk5 activator, the 23 kDa subunit of tau protein kinase II: its sequence and developmental change in brain. *FEBS Lett.,* 1994, vol. 355, 35-40
- **UCHIDA et al.** An isoform of the neuronal cyclin-dependent kinase 5 (Cdk5) activator. *J. Biol. Chem.,* 1995, vol. 270, 26897-26903
- **TANG et al.** The cyclin-dependent kinase 5 activator, p39, is expressed in stripes in the mouse cerebellum. *Neuroscience,* 2003, vol. 118, 323-334
- **JEONG et al.** The role of cyclin-dependent kinase 5 in brain development and degeneration Tsai L.H. *Cell. Mol. Biol. Lett.,* 2003, vol. 8, 546, 547
- An Unusual Memberii of the Cdk Family: Cdk5. *Cell Mol Neurobiol,* 2008, vol. 28, 351-369
- **DHARIWALA F.A. ; RAJADHYAKSHA M.S.** Callosal axon guidance defects in p35 (-/-) mice. *J. Comp. Neurol.,* 1999, vol. 415, 218-229
- Cyclin-Dependent Kinase Inhibitors Attenuate Protein Hyperphosphorylation, Cytoskeletal Lesion Formation, and Motor Defects in Niemann-Pick Type C Mice. *American Journal of Pathology,* 2004, vol. 165 (3), 843-853
- **ZHANG M. et al.** p35/ Cdk5 pathway mediates soluble amyloid-beta peptide-induced tau phosphorylation in vitro. *J. Neurosci. Res.,* 2002, vol. 69, 362-372
- **IMAHORI K. ; UCHIDA T.** Physiology and pathology of tau protein kinases in relation to Alzheimer's disease. *J. Biochem. (Tokyo),* 1997, vol. 121, 179-188
- **NOBLE W.** Cdk5 Is a Key Factor in Tau Aggregation and Tangle Formation In Vivo. *Neuron,* 2003, vol. 38, 555-565
- **CRUZ J.C.** Aberrant Cdk5 Activation by p25 Triggers Pathological Events Leading to Neurodegeneration and Neurofibrillary Tangles. *Neuron,* 2003, vol. 40, 471-483
- **WEN, Y.** Interplay between Cyclin-Dependent Kinase 5 and Glycogen Synthase Kinase 3β Mediated by Neuregulin Signaling Leads to Differential Effects on Tau Phosphorylation and Amyloid Precursor Protein Processing. *J. Neurosci.,* 2008, vol. 28, 2624-2632
- **TSAI L.H., ; LEE M.S. ; CRUZ J.** Cdk5, a therapeutic target for Alzheimer's disease?. *Biochimica et Biophysica Acta,* 2004, vol. 1697, 137-142
- **TSAI L.H. ; LEE M.S. ; CRUZ J.** Cdk5, a therapeutic target for Alzheimer's disease?. *Biochimica et Biophysica Acta,* 2004, vol. 1697, 137-142
- **BAJAJ.** Cyclin-dependent kinase-5 is associated with lipofuscin in motor neurones in amyotrophic lateral sclerosis. *Neurosci. Lett.,* 1998, vol. 245, 45-48
- **BU B.** Niemann-Pick disease type C yields possible clue for why cerebellar neurons do not form neurofibrillary tangles. *Neurobiol Dis,* 2002, vol. 11, 285-297

- **RAJGOPAL Y. ; VEMURI M.C.** Ethanol induced changes in cyclin-dependent kinase-5 activity and its activators, P35, P67 (Munc-18) in rat brain. *Neurosci. Lett.,* 2001, vol. 308, 173-176
- **BRION J.P. ; COUCK A.M.** Cortical and brain-stem-type Lewy bodies are immunoreactive for the cyclin-dependent kinase 5. *Am. J. Pathol.,* 1995, vol. 147, 1465-1476
- **SMITH P.D.** Cyclin-dependent kinase 5 is a mediator of dopaminergic neuron loss in a mouse model of Parkinson's disease. *Proc Natl Acad Sci U S A,* 2003, vol. 100, 13650-13655
- **BORGHI R.** Increase of cdk5 is related to neurofibrillary pathology in progressive supranuclear palsy. *Neurology,* 2002, vol. 58, 589-592
- **HAN D.** Familial FTDP-17 missense mutations inhibit microtubule assembly-promoting activity of tau by increasing phosphorylation at Ser202 in vitro. *J Biol Chem.,* 2009, vol. 284 (20), 13422-33
- **SULTANA R. ; BUTTERFIELD D.A.** Regional expression of key cell cycle proteins in brain from subjects with amnestic mild cognitive impairment. *Neurochem Res.,* 2007, vol. 32 (4-5), 655-62
- **POLLONINI G.** Abnormal expression of synaptic proteins and neurotrophin-3 in the Down syndrome mouse model Ts65Dn. *Neuroscience,* 2008, vol. 156 (1), 99-106
- **PAOLETTI P.** Dopaminergic and glutamatergic signaling crosstalk in Huntington's disease neurodegeneration: the role of p25/cyclin-dependent kinase 5. *J Neurosci.,* 2008, vol. 28 (40), 10090-101
- **SCHNEIDER A.** Hyperphosphorylation and aggregation of tau in experimental autoimmune encephalomyelitis. *J Biol Chem.,* 2004, vol. 279 (53), 55833-9
- **OSUGA H.** Cyclin-dependent kinases as a therapeutic target for stroke. *Proc Natl Acad Sci USA,* 2000, vol. 97, 10254-10259
- **WANG J.** Cdk5 activation induces hippocampal CA1 cell death by directly phosphorylating NMDA receptors. *Nature Neuroscience,* 2003, vol. 6, 1039-1047
- **SHELTON S.B. ; JOHNSON G.V.W.** Cyclin-dependent kinase-5 in neurodegeneration. *J Neurochem,* 2004, vol. 88 (6), 1313-1326
- **BIBB J.A.** Effects of chronic exposure to cocaine are regulated by the neuronal protein Cdk5. *Nature,* 2001, vol. 410, 376-380
- **CHEN J.** Induction of Cyclin-Dependent Kinase 5 in the Hippocampus by Chronic Electroconvulsive Seizures: Role of ΔFosB. *J. Neurosci.,* 2000, vol. 20, 8965-8971
- **SEN A.** The potential role of cyclin-dependent kinase 5 in focal cortical dysplasia. *Dev Neurosci.,* 2008, vol. 30 (1-3), 96-104
- **GREEN S.** Alterations in Cyclin-Dependent Protein Kinase 5 (CDK5) Protein Levels, Activity and Immunocytochemistry in Canine Motor Neuron Disease. *J. Neuropathol. Exp. Neurol.,* 1998, vol. 57 (11), 991-1099
- **NAKANO S.** Aberrant expression of cyclin-dependent kinase 5 in inclusion body myositis. *Neurology,* 1999, vol. 53, 1671-1676
- **NAKAMURA S.** Cyclin-dependent kinase 5 and mitogen-activated protein kinase in glial cytoplasmic inclusions in multiple system atrophy. *J. Neuropathol. Exp. Neurol.,* 1998, vol. 57, 690-698
- **FU W. Y.** Induction of Cdk5 activity in rat skeletal muscle after nerve injury. *Neuroreport.,* 2002, vol. 13, 243-247
- **BIGNANTE E.A.** Previous stress exposure enhances both anxiety-like behaviour and p35 levels in the basolateral amygdala complex: Modulation by midazolam. *Eur Neuropsychopharmacol.,* 2010, vol. 20 (6), 388-397
- **XI Z. Q.** Gene expression analysis on anterior temporal neocortex of patients with intractable epilepsy. *Synapse,* 2009, vol. 63 (11), 1017-1028
- **PARESA N.** Phosphorylation of Prion Protein at Serine 43 Induces Prion Protein Conformational Change. *J Neurosci.,* 2009, vol. 29 (27), 8743-8751
- **CHEUNG K.J.** Tyrosine hydroxylase expression and Cdk5 kinase activity in ataxic cerebellum. *Mol Cell Biochem.,* 2008, vol. 318 (1-2), 7-12
- **MIZUNO K.** Expression of p25 impairs contextual learning but not latent inhibition in mice. *Neuroreport.,* 2006, vol. 17 (18), 1903-5
- **TEJ K.** Cdk5: A New Player in Pain Signaling. *Cell Cycle,* 2006, vol. 6, 585-588
- **YASUDA H.** New trend in pathogenesis of diabetic neuropathy. *Rinsho Shinkeigaku.,* 1999, vol. 39 (1), 87-9
- **WEI F. Y. ; TOMIZAWA K.** Cyclin-dependent kinase 5 (Cdk5): a potential therapeutic target for the treatment of neurodegenerative diseases and diabetes mellitus. *Mini Rev Med Chem.,* 2007, vol. 7 (10), 1070-4
- **COGHLAN.** Selective small molecule inhibitors of glycogen synthase kinase-3 modulate glycogen metabolism and gene transcription. *Chemistry & Biology,* 2000, vol. 7 (10), 793-803
- **KIM, L. ; KIMMEL, A.R.** GSK3, a master switch regulating cell-fate specification and tumorigenesis. *Curr. Opinion Genetics Dev.,* 2000, vol. 10 (5), 508-514
- **ÁVILA, J.** Role of tau protein in both physiological and pathological conditions. *Physiol. Rev.,* 2004, vol. 84, 361-84
- **JUHASZOVA M.** Role of glycogen synthase kinase-3beta in cardioprotection. *Circ Res.,* 2009, vol. 104 (11), 1240-52

- **MIURA T. ; MIKI T.** GSK-3beta, a therapeutic target for cardiomyocyte protection. *Circ J.,* 2009, vol. 73 (7), 1184-92
- **ELDAR-FINKELMAN, H.** Glycogen synthase kinase 3: an emerging therapeutic target. *Trends. Mol. Med.,* 2002, vol. 8, 126-32
- **ASENJO, A.** Residues in human respiratory syncytial virus P protein that are essential for its activity on RNA viral synthesis. *Virus Res.,* 2008, vol. 132, 160-73
- **HERNANDEZ F.** The role of GSK3 in Alzheimer disease. *Brain Res Bull.,* 2009, vol. 80 (4-5), 248-50
- **MASAHIRO N. ; HIDEAKI H.** Glycogen synthase kinase-3beta is associated with Parkinson's disease. *Neuroscience Letters,* 2009, vol. 449 (2), 103-107
- **SCHAFFER, B.** Association of GSK3B with Alzheimer disease and frontotemporal dementia. *Arch. Neurol.,* 2008, vol. 65, 1368-74
- **BUÉE, L.** Tau protein isoforms, phosphorylation and role in neurodegenerative disorders. *Brain Research Reviews,* 2000, vol. 33, 95-130
- **DEWHURST S.** Glycogen synthase kinase 3 beta (GSK-3 beta) as a therapeutic target in neuroAIDS. *J Neuroimmune Pharmacol.,* 2007, vol. 2 (1), 93-96
- **CARMICHAEL J.** Glycogen synthase kinase-3beta inhibitors prevent cellular polyglutamine toxicity caused by the Huntington's disease mutation. *J Biol Chem.,* 2002, vol. 277 (37), 33791-8
- **TANJI K.** Glycogen synthase kinase-3beta phosphorylates synphilin-1 in vitro. *Neuropathology.,* 2003, vol. 23 (3), 199-202
- **ROEW M.K.** GSK-3 is a viable potential target for therapeutic intervention in bipolar disorder. *Neurosci Biobehav Rev.,* 2007, vol. 31 (6), 920-931
- **LI X.** Glycogen Synthase Kinase-3β, mood stabilizers, and neuroprotection. *Bipolar Disord.,* 2002, vol. 4 (2), 137-144
- **WADA A.** Lithium and neuropsychiatric therapeutics: neuroplasticity via glycogen synthase kinase-3beta, beta-catenin, and neurotrophin cascades. *J Pharmacol Sci.,* 2009, vol. 110 (1), 14-28
- **KOROS E. ; DORNER-CIOSSEK C.** The role of glycogen synthase kinase-3beta in schizophrenia. *Drug News Perspect.,* 2007, vol. 20 (7), 437-45
- **LOVESTONE S.** Schizophrenia as a GSK-3 dysregulation disorder. *Trends Neurosci.,* 2007, vol. 30 (4), 142-9
- **CHEN G.** The mood-stabilizing agent valproate inhibits the activity of glycogen synthase kinase-3. *J Neurochem.,* 1999, vol. 72 (3), 1327-30
- **JOPE R. S. ; ROH M.S.** Glycogen synthase kinase-3 (GSK3) in psychiatric diseases and therapeutic interventions. *Curr Drug Targets.,* 2006, vol. 7 (11), 1421-34
- **BEAULIEU J.M.** Role of GSK3 beta in behavioral abnormalities induced by serotonin deficiency. *Proc Natl Acad Sci U S A.,* 2008, vol. 105 (4), 1333-8
- **BEAULIEU J.M.** Lithium antagonizes dopamine-dependent behaviors mediated by an AKT/glycogen synthase kinase 3 signaling cascade. *Proc Natl Acad Sci U S A.,* 2004, vol. 101 (14), 5099-104
- **LIU F.** Overexpression of Dyrk1A contributes to neurofibrillary degeneration in Down syndrome. *FASEB J.,* 2008, vol. 22 (9), 3224-33
- **DUGO L.** Glycogen synthase kinase 3beta as a target for the therapy of shock and inflammation. *Shock.,* 2007, vol. 27 (2), 113-23
- **SASAKI C.** Different expression of glycogen synthase kinase-3beta between young and old rat brains after transient middle cerebral artery occlusion. *Neurol Res.,* 2001, vol. 23 (6), 588-92
- **BEUREL E.** Innate and adaptive immune responses regulated by glycogen synthase kinase-3 (GSK3). *Trends Immunol.,* 2010, vol. 31 (1), 24-31
- **DE SARNO P.** Lithium prevents and ameliorates experimental autoimmune encephalomyelitis. *J. Immunol.,* 2008, vol. 181 (1), 338-45
- **WATASE K.** Lithium therapy improves neurological function and hippocampal dendritic arborization in a spinocerebellar ataxia type 1 mouse model. *PLoS Med,* 2007, vol. 4 (5), 836-847
- **HAN F.** Accumulation of beta-amyloid in the brain microvessels accompanies increased hyperphosphorylated tau proteins following microsphere embolism in aged rats. *Neuroscience.,* 2008, vol. 153 (2), 414-27
- **YANG W.** Upregulation of GSK3β expression in frontal and temporal cortex in ALS with cognitive impairment (ALSci). *Brain Res.,* 2008, vol. 1196, 131-139
- **DIII, J.** Inactivation of glycogen synthase kinase 3 promotes axonal growth and recovery in the CNS. *J. Neurosci.,* 2008, vol. 28, 8914-28
- **COHEN P.** The role of protein phosphorylation in human health and disease. *Eur J Biochem.,* 2001, vol. 268 (19), 5001-10
- **CUZZOCREA, S.** Glycogen synthase kinase-3b inhibition attenuates the degree of arthritis caused by type II collagen in the mouse. *Clin. Immunol.,* 2006, vol. 120, 57-67
- **HU, X.** IFN-g suppresses IL-10 production and synergizes with TLR2 by regulating GSK3 and CREBIAP-1 proteins. *Immunity,* 2006, vol. 24, 563-574
- **DUGO, L.** GSK-3b inhibitors attenuate the organ injury/dysfunction caused by endotoxemia in the rat. *Crit. Care Med.,* 2005, vol. 33, 1903-1912
- **BENTLEY J.K.** Airway smooth muscle hyperplasia and hypertrophy correlate with glycogen synthase kinase-3(beta) phosphorylation in a mouse model of asthma. *Am J Physiol Lung Cell Mol Physiol.,* 2009, vol. 296 (2), L176-84

- **EVENSON, A.R.** GSK-3b inhibitors reduce protein degradation in muscles from septic rats and in dexamethasone treated myotubes. Int. *J. Biochem. Cell. Biol.,* 2005, vol. 37, 2226-2238
- **WHITTLE, B.J.** Reduction of experimental colitis in the rat by inhibitors of glycogen synthase kinase-3b. *Br. J. Pharmacol.,* 2006, vol. 147, 575-582
- **DUGO, L.** Glycogen synthase kinase-3b inhibitors protect against the organ injury and dysfunction caused by hemorrhage and resuscitation. *Shock,* 2006, vol. 25, 485-491
- **GONG R.** Glycogen synthase kinase 3beta: a novel marker and modulator of inflammatory injury in chronic renal allograft disease. *Am J Transplant.,* 2008, vol. 8 (9), 1852-63
- **LENZ, S.P.** Lithium chloride enhances survival of NZB/W lupus mice: influence of melatonin and timing of treatment. *Int. J. Immunopharmacol.,* 1995, vol. 17, 581-592
- **HARDT, S.E. ; SADOSHIMA, J.** Glycogen synthase kinase-3beta: a novel regulator of cardiac hypertrophy and development. *Circ. Res.,* 2002, vol. 90, 1055-63
- **BOWES A.J.** Valproate attenuates accelerated atherosclerosis in hyperglycemic apoE-deficient mice: evidence in support of a role for endoplasmic reticulum stress and glycogen synthase kinase-3 in lesion development and hepatic steatosis. *Am J Pathol.,* 2009, vol. 174 (1), 330-42
- **BADORFF C.** Fas receptor signaling inhibits glycogen synthase kinase 3β and induces cardiac hypertrophy following pressure overload. *J. Clin. Invest.,* 2002, vol. 109 (3), 373-381
- **MA X.** Delayed Re-endothelialization with Rapamycin-coated Stents is Rescued by the Addition of a Glycogen Synthase Kinase 3 Beta Inhibitor. *Cardiovasc Res.,* 2010
- **GALLICCHIO, V. S.** Lithium and the Cell. Academic, 1991, 185-198
- **WAGMAN A.S.** Discovery and development of GSK3 inhibitors for the treatment of type 2 diabetes. *Curr Pharm Des.,* 2004, vol. 10 (10), 1105-37
- **LYUBIMOVA A.** Neural Wiskott-Aldrich syndrome protein modulates Wnt signaling and is required for hair follicle cycling in mice. *J Clin Invest.,* 2010, vol. 120 (2), 446-56
- **WU C.H.** Glycogen synthase kinase-3 regulates the phosphorylation of severe acute respiratory syndrome coronavirus nucleocapsid protein and viral replication. *J Biol Chem.,* 2009, vol. 284 (8), 5229-39
- **XU C.M.** Glycogen synthase kinase 3beta in the nucleus accumbens core mediates cocaine-induced behavioral sensitization. *J Neurochem.,* 2009, vol. 111 (6), 1357-68
- **WANG F.S.** Inhibition of glycogen synthase kinase-3beta attenuates glucocorticoid-induced bone loss. *Life Sci.,* 2009, vol. 85 (19-20), 85-92
- **YUSKAITIS C.J.** Lithium ameliorates altered glycogen synthase kinase-3 and behavior in a mouse model of fragile X syndrome. *Biochem Pharmacol.,* 2010, vol. 79 (4), 632-46
- **WANG WH.** Increased expression of the WNT antagonist sFRP-1 in glaucoma elevates intraocular pressure. *J Clin Invest.,* 2008, vol. 118 (3), 1056-64
- **COHEN, P. ; GOEDERT, M.** GSK3 inhibitors: development end therapeutic potential. *Nature Reviews,* 2004, vol. 3, 479-487
- **HERNÁNDEZ, F.** GSK3 Inhibitors and Disease. *Mini-Reviews in Medicinal Chemistry,* 2009, vol. 9 (9), 1024-1029
- **MEDINA, M. ; CASTRO, A.** Glycogen synthase kinase-3 (GSK-3) inhibitors reach the clinic. *Curr. Opin. Drug Discov. Develop.,* 2008, vol. 11 (4), 533-543
- Glycogen Synthase Kinase 3 (GSK-3) and its inhibitors. John Wiley & Sons, Inc, 2006
- **LECLERC, S.** Indirubins inhibit glycogen synthase kinase-3 beta and CDK5/p25, two protein kinases involved in abnormal tau phosphorylation in Alzheimer's disease. A property common to most cyclin-dependent kinase inhibitors?. *J. Biol. Chem.,* 2001, vol. 276, 251-60
- **SMITH, D.** 3-Anilino- 4-arylmaleimides: potent and selective inhibitors of glycogen synthase kinase-3 (GSK-3). *Bioorg. Med. Chem. Lett.,* 2001, vol. 11, 635-9
- **WITHERINGTON, J.** 5-arylpyrazolo[3,4-b]pyridazines: potent inhibitors of glycogen synthase kinase-3 (GSK-3). *Bioorg. Med. Chem. Lett.,* 2003, vol. 13, 1581-4
- **LEOST, M.** Paullones are potent inhibitors of glycogen synthase kinase-3beta and cyclin-dependent kinase 5/p25. *Eur. J. Biochem.,* 2000, vol. 267, 5983-94
- **BHAT, R.** Structural insights and biological effects of glycogen synthase kinase 3-specific inhibitor AR-A014418. *J. Biol. Chem.,* 2003, vol. 278, 45937-45
- **MARTINEZ, A.** First non-ATP competitive glycogen synthase kinase 3 beta (GSK-3beta) inhibitors: thiadiazolidinones (TDZD) as potential drugs for the treatment of Alzheimer's disease. *J. Med. Chem.,* 2002, vol. 45, 1292-9
- *Org. Biomol. Chem.,* 2008, vol. 6, 2765-2771
- **KROGSGAARD-LARSEN et al.** Textbook of Drug design and Discovery. Taylor & Francis, April 2002
- **CAPON R.J. ; PENG C. ; DOOMS C.** Trachycladindoles A-G: cytotoxic heterocycles from an Australian marine sponge, Trachycladus laevispirulifer. *Organic & Biomolecular Chemistry,* 2008, vol. 6, 2765-2771